# EUROPEAN PATENT SPECIFICATION

(11) **EP 3 759 500 B1**
(45) Date of publication and mention of the grant of the patent: **11.06.2025**
(21) Application number: 19756643.3
(22) Date of filing: 14.02.2019
(51) Int. Cl.: G01N 33/94, A61K 31/397, A61P 3/06, A61K 9/14

(54) **EZETIMIBE METABOLITE AS A NON-INVASIVE BIOMARKER FOR NON-ALCOHOLIC STEATOHEPATITIS (NASH)**
EZETIMIB-METABOLIT ALS NICHT-INVASIVER BIOMARKER FÜR NICHTALKOHOLISCHE STEATOHEPATITIS (NASH)
MÉTABOLITE D'ÉZÉTIMIBE EN TANT QUE BIOMARQUEUR NON INVASIF POUR LA STÉATOHÉPATITE NON ALCOOLIQUE (SHNA)

(30) Priority: 26.02.2018 US 201862635273 P
(43) Date of publication of application: 06.01.2021
(73) Proprietor: Arizona Board of Regents on behalf of the University of Arizona, Tucson, AZ 85721 (US)
(72) Inventor: CHERRINGTON, Nathan, J., Tucson, AZ 85721 (US)
(74) Representative: Brand Murray Fuller LLP
(86) International application number: PCT/US2019/018038
(87) International publication number: WO 2019/164749

(56) References cited:
- WO-A1-2017/065206
- US-B2- 7 314 720
- RHIANNON N. HARDWICK ET AL: "Molecular Mechanism of Altered Ezetimibe Disposition in Nonalcoholic Steatohepatitis", DRUG METABOLISM AND DISPOSITION, vol. 40, no. 3, 23 November 2011 (2011-11-23), US, pages 450 - 460, XP055632255, ISSN: 0090-9556, DOI: 10.1124/dmd.111.041095
- ANIKA L. DZIERLENGA ET AL: "Misregulation of membrane trafficking processes in human nonalcoholic steatohepatitis", JOURNAL OF BIOCHEMICAL AND MOLECULAR TOXICOLOGY, vol. 32, no. 3, 17 January 2018 (2018-01-17), US, pages e22035, XP055632259, ISSN: 1095-6670, DOI: 10.1002/jbt.22035
- HARDWICK ET AL.: "Molecular Mechanism of Altered Ezetimibe Disposition in Nonalcoholic Steatohepatitis", DRUG METAB DISPOS., vol. 40, no. 3, 2012, pages 450 - 460, XP055632255
- DZIERLENGA ET AL.: "Misregulation of membrane trafficking processes in human nonalcoholic steatohepatitis", J BIOCHEM MOL TOXICOL., vol. 32, no. 3, 17 January 2018 (2018-01-17), pages e22035, XP055632259
- ADINOLFI ET AL.: "NAFLD and NASH in HCV Infection: Prevalence and Significance in Hepatic and Extrahepatic Manifestations", INT J MOL SCI., vol. 17, no. 6, 2016, pages 803, XP055632263

## Description

### BACKGROUND OF THE INVENTION

### Field of the Invention

The present invention relates to an *in vitro* method of diagnosing a hepatic disorder in a human subject at risk of the hepatic disorder, to an *in vitro* method of monitoring progression of a hepatic disorder in a human subject, to an *in vitro* method of monitoring effectiveness of a treatment for a hepatic disorder in a human subject and an *in vitro* method of delineating patients with non-alcoholic steatohepatitis (NASH) from those with simple steatosis. Due to two key molecular changes in disease progression from simple steatosis to NASH, the use of ezetimibe-glucuronide (EZE-Gluc) as a diagnostic biomarker could shift clinical practice paradigms and enable the establishment of a non-invasive, specific, and selective biomarker for identifying patients with NASH.

Non-alcoholic Fatty Liver Disease (NAFLD) is the most prevalent chronic liver disease, affecting 25% people worldwide and 64 million people in the US, which caused $103 billion of economic burden on the US healthcare system annually. Although first described almost 30 years ago, the true scope of NAFLD and its clinical burden has only recently been understood. The initial stage of NAFLD is simple steatosis, which is characterized by microvesicular fat deposition and is often referred to as non-alcoholic fatty liver (NAFL). Although the spectrum of NAFLD was originally thought to be benign, approximately 10% of patients with steatosis progress to non-alcoholic steatohepatitis (NASH), which has significant clinical implications. NASH is histologically characterized by macrovesicular steatosis, lobular inflammation, and hepatocellular damage including Mallory's hyaline, ballooning degeneration, necrosis, and fibrosis (portal and zone 3). As well, NASH is the most common cause of cryptogenic cirrhosis, and it is estimated that 30 to 50% of NASH patients will progress to cirrhosis within 10 years. Because of this, NASH is reported to be the underlying cause of 10% of liver transplants. Additionally, NASH is responsible for an estimated 13% of all hepatocellular carcinoma cases. Clearly, due to the increased risk of developing more severe diseases, a critical need exists for a diagnostic tool that allows the definitive and reliable identification of patients with NASH.

In addition to innate liver-related morbidity and mortality, NASH patients might also be at increased risk for adverse drug reactions, and NASH has been recognized as an additive factor in other liver injuries (toxicants, viruses). It has been asserted that changes in hepatic gene expression and resulting altered pharmacokinetics specific to NASH may partially explain the increased risk for chemical toxicities. Because the identified changes in hepatic gene expression might lead to an increased risk of toxic events in NASH patients, it is proposed these gene expression changes can be utilized to develop a novel method of identifying NASH patients.

### Description of Related Art Including Information Disclosed

Development of reliable diagnostic techniques of NAFL and NASH has been an evolving process as our understanding of these diseases has progressed. The heterogeneity of methods used for diagnosis has significantly impacted the prevalence estimates for the different stages of NAFLD and creates a major roadblock in efforts to elucidate all of the clinical ramifications of this disease. One of the chief problems is that most patients with NAFLD are asymptomatic and can harbor this silent time bomb for years without knowing they have the disease.

The simplest non-invasive diagnostic technique would be a blood borne biomarker that can distinguish NAFL from NASH. Regrettably, for many patients, diagnosis occurs following findings of elevated aminotransferases combined with features of metabolic syndrome and other more serious pathological indications of advanced liver disease. Discovery of the disease at this later stage can complicate the management of the disease and render treatment more difficult. Unfortunately, relying on liver enzyme levels is not effective because normal serum aminotransferase tests are often noted in patients with both steatosis and NASH. Indeed, reports indicate that two-thirds of NASH patients might have normal aminotransferase levels at any given time. The accuracy of NASH diagnosis by serum ALT was examined in women undergoing gastric bypass surgery. Two different reference laboratory cutoffs for "normal" ALT levels were examined, the previous guideline of 30 U/L, and a lower level of 19 U/L, which was suggested to aid in the diagnosis of NAFLD. Importantly, the diagnostic utility of serum ALT remained poor, even at the new lower cutoff. The sensitivity and specificity of serum ALT levels were found to be 42% and 80% (ALT > 30 U/L) respectively, versus 74% and 42% (ALT > 19 U/L), respectively.

Another plasma biomarker that initially held promise as a non-invasive NASH diagnostic was plasma caspase-generated cytokeratin-18 fragments (CK-18), but ultimately CK-18 proved to be of little clinical value. Other blood borne biomarkers including C-reactive protein and γ-glutamyl transferase are not specific enough due to the fact that these proteins are also related to many other inflammatory and disease-related processes. Thus, the results of prior studies substantiate the need for a more effective diagnostic measure for NAFLD, especially the NASH stage.

Imaging techniques such as ultrasonography (US), computerized tomographic (CT) scanning, and magnetic resonance imaging (MRI) have been used successfully to diagnose NAFL. Of these methods, US is preferred because of lower associated costs and accessibility, although it has several limitations. For example, sensitivity is high (80%) in patients with >30% steatosis, but drops to 55% with steatosis < 19%. Similarly, in morbidly obese patients, sensitivity drops to 49%. Similar decreases occur with respect to specificity. Additionally, US is unable to quantitate the degree of lipid accumulation. Both MRI and CT scans provide an accurate quantification of steatosis, but the higher associated costs can be prohibitive. A more recent technique, transient elastography, measures liver stiffness and might be effective in assessing the level of hepatic fibrosis in later stages of NAFLD. However, a recent study found an increased failure rate in overweight and obese patients, which might limit the effectiveness of this technique in NAFLD. Ultimately, the most important limitation of each of these imaging methods is the inability to distinguish between steatosis and NASH.

Liver histology remains the gold standard for NASH diagnosis, as it is able to distinguish steatosis, fibrosis, and inflammation. Importantly, this ability to stage and grade NAFLD allows the differentiation between NAFL and NASH. The principle features of NASH include macrovesicular steatosis, lobular inflammation, and ballooning degeneration. The histological diagnosing criteria have been standardized by the implementation of the NAFLD activity score (NAS), developed by the NASH Clinical Research Network. NAS consists of an unweighted sum of scores for steatosis, lobular inflammation, and hepatocellular ballooning, in which scores ≥ 5 are classified as NASH, and scores < 3 are not-NASH. In addition to these criteria, liver biopsies also reveal the degree of liver damage and any changes in overall liver architecture. Other staining markers can distinguish NAFL from NASH including perilipin-1 (PLIN1) and PLIN2 lipid droplet proteins; however, these stains still require the performance of an invasive liver biopsy.

Aside from the invasive nature of liver biopsies, there are other significant clinical limitations to this procedure. Studies have indicated significant risks and complications associated with liver biopsies, including pain, major bleeding, and death. It has been estimated that 1 to 3% of patients might require hospitalization following a liver biopsy, and in at least one study, biopsy-related mortality occurred in over 1% of biopsies. Other identified limitations of liver biopsies include the subjective nature of the histological analysis and the possibility of sampling error due to the relatively small sample size. Unfortunately, despite these concerns, liver biopsy remains the only proven, reliable method of diagnosing NASH.

Previous studies have utilized a methionine- and choline-deficient (MCD) diet fed mammalian model to reflect human non-alcoholic steatohepatitis (NASH). For example, in Hardwick et al. Molecular Mechanism of Altered Ezetimibe Disposition in Nonalcoholic Steatohepatitis, Drug Metab Dispos. 2012 Mar; 40(3): 450-460, herein referred to as the Hardwick article, an MCD mammalian model was employed in order to demonstrate the possibility of potential adverse drug reactions in NASH patients. The purpose of the Hardwick article was to identify drugs that may have potential toxicities in NASH, and to identify NASH as a potential variable in the inter-individual differences in drug metabolism and disposition that lead to adverse drug reactions in at-risk patients. A number of differences in that study make the altered disposition and potential toxicity observed in the MCD animal model distinct from the possible use of that drug as a biomarker to predict human disease progression.

First is that the MCD rodent model fails to accurately depict the human NASH metabolic abnormalities found in the pathogenesis of the human disease. For example, the MCD rodent model does not lead to insulin sensitivity or any of the other symptoms of the metabolic syndrome, and a well-documented side effect in the MCD model is weight loss. Thus, the MCD model is a very poor model for translational application to obese human populations since weight loss in the MCD model is common and well-documented, and other symptoms of the metabolic syndrome are absent. NASH patients are most often obese, have a difficult time losing weight, and suffer from multiple co-morbidities associated with the metabolic syndrome. Additionally, the biochemical events associated with disease progression in the MCD rodent model are dramatically different than human NASH progression, including glutathione depletion and numerous differences in amino acid and bile acid metabolite profiles that regulate liver health.

Second is the well documented difference between species in the hepatobiliary disposition of glucuronide conjugates, where human favors renal elimination much more than rodent species. Several other drugs including acetaminophen that are similarly glucuronidated are known to undergo renal elimination into the urine of humans at a much greater rate than in rodents. Thus, observations of glucuronide metabolite disposition using the rodent models may not be reflective of human disposition. Therefore, it would not be logical to use the results of the MCD rodent model to directly extrapolate to the human condition of NASH or for diagnosing a hepatic disorder in a human since the MCD rodent model does not translate to the obese population.

Third, the use of an exogenous molecule as a biomarker is not standard in diagnosing disease progression. Studies of altered disposition in diseased patients are associated with toxicity where the altered disposition is the actual mechanism that makes these patients more susceptible. Additionally, the typical strategy for diagnosing disease includes measurement of an endogenous protein or compound that has been modified (A1C) or is present in higher concentrations or places where it is not normally found (PSA in the blood, etc.). Sampling for these standard tests can be conducted at any time because the endogenous protein or compound being tested is inherently present in the diseased patient. Exogenous biomarkers, on the other hand, must first introduce a foreign compound to a patient and then wait for some period of time while some process related to disease progression occurs. Exogenous biomarkers also have the additional safety concerns of introducing foreign compounds to the patient. For these significant reasons and others not listed here, a person having ordinary skill in the art would understand that the limitations of the Hardwick article's adverse drug reaction study using a rodent MCD model would not determine the next logical step of the prior art to be the current methodology.

WO2017/065206 discloses a marker for non-alcoholic steatohepatitis, comprising a procollagen C-endopeptidase enhancer (PCPE-1) gene or a PCPE-1 protein.

US7314720 discloses a method consisting of detecting and quantifying, *in vitro* in a hepatic tissue sample, the levels of a protein which can be used as a NASH molecular marker and which is selected from apolipoprotein A1, sub-unit beta of the mitochondrial ATPase, leukotriene A4 hydrolase, keratin 18, guanidine acetate N-methyltransferase, superoxide dismutase, albumin, antioxidant protein 2 (isoform 1), prohibitin 1, methionine adenosyl transferase, long-chain acyl CoA dehydrogenase, selenium binding protein, antioxidant protein 2 (isoform 2), and combinations of same. The method further involves comparing the results obtained with the normal values of said proteins in healthy hepatic tissue and can be used to diagnose NASH and/or to assess a patient's potential risk of developing NASH.

BRIEF SUMMARY OF THE INVENTION The invention is defined in the appended claims.

It is an objective of the present invention to provide an *in vitro* method that allows for diagnosing and monitoring a hepatic disorder as specified in the independent claims. In particular, the method is used to distinguish patients with non-alcoholic steatohepatitis (NASH) from those with simple steatosis. Embodiments of the invention are given in the dependent claims. Embodiments of the present invention can be freely combined with each other if they are not mutually exclusive.

The present invention relates to a non-invasive, *in vitro,* method of delineating patients with non-alcoholic steatohepatitis (NASH) from those with steatosis. The initial stage of non-alcoholic fatty liver disease (NAFLD) is simple steatosis, which is characterized by microvesicular fat deposition and is often referred to as non-alcoholic fatty liver (NAFL). Although the spectrum of NAFLD was originally thought to be benign, approximately 10% of patients with steatosis progress to non-alcoholic steatohepatitis (NASH), which has significant clinical implications. Due to two key molecular changes in disease progression from simple steatosis to NASH, the use of ezetimibe-glucuronide (EZE-Gluc) as a diagnostic biomarker could shift clinical practice paradigms and enable the establishment of a non-invasive, specific, and selective biomarker for identifying patients with NASH.

In some aspects, the present invention features *in vitro* methods of diagnosing a hepatic disorder (e.g., NASH) in a human subject. The methods comprise (a) determining an amount of EZE and/or ezetimibe-glucuronide (EZE-Gluc) in a plasma sample and/or a urine sample that was obtained from the human subject some time after the human subject has been administered EZE, (b) comparing the amount of EZE and/or EZE-Gluc in one or both of the plasma sample and the urine sample to a control (e.g., EZE-Gluc in the subject compared to EZE-Gluc in the control; EZE in the subject compared to EZE in the reference), and (c) identifying the human subject as having a hepatic disorder if the amount of EZE and/or EZE-Gluc in one or both of the plasma sample and the urine sample is elevated relative to the control (e.g., higher plasma EZE-Gluc in the human subject compared to plasma EZE-Gluc level in the control; higher urine EZE-Gluc in the human subject compared to urine EZE-Gluc level in the control; higher plasma EZE in the human subject compared to plasma EZE level in the control).

One of the unique and inventive technical features of the present invention is the use of the altered disposition of the glucoronidated metabolite of EZE (EZE-Gluc) as a metabolic biomarker to diagnose and monitor a hepatic disorder in subjects who have been administered EZE. Without wishing to limit the invention to any theory or mechanism, it is believed that the technical feature of the present invention advantageously allows for the use of EZE-Gluc as a diagnostic biomarker of a hepatic disorder. None of the presently known prior references or work has the unique inventive technical feature of the present invention.

In addition to the aforementioned reasons as to why the altered disposition and potential toxicity observed in the MCD rodent model is distinct from the possible use of that drug as a biomarker to predict human disease progression, prior references teaches away from the present invention. For example, altered drug disposition is typically a mechanism of toxicity, not a biomarker. More drug staying in the blood or urine typically causes those patients to be at greater risk of toxicity by that drug. For example, animals with methionine and choline deficient diets treated with either morphine or methotrexate, which are similarly handled by drug transporters, had altered pharmacologic and toxicologic profiles than healthy controls. Therefore, the prior art discourages the use of exogenous biomarkers for hepatic disorders.

Furthermore, the inventive technical features of the present invention contributed to a surprising result. A surprising and unexpected result can occur in patients who have been administered EZE. Without wishing to limit the invention to a particular theory or mechanism, there may be significantly higher concentrations of the metabolized compound EZE-Gluc found in the plasma and urine of NASH patients, with decreased biliary elimination, as compared to patients with healthy or steatotic livers. For example, in patients administered acetaminophen (APAP), which is similarly metabolized to a glucuronide conjugate, much lower levels are detected in the blood and plasma compared to EZE, therefore indicating that the metabolized forms detected in plasma and urine would not make for a good biomarker. In fact, EZE-Gluc was surprisingly found to be a more specific and selective substrate for the molecular mechanisms that are altered in NASH than was APAP-Gluc, with up to a 100-fold increase in plasma and urine. One of ordinary skill in the art would not be able to predict this result.

One of the benefits of the particular drug that we are using is that it is specific for the transporters that are altered by NASH, e.g., organic anion transporter proteins (OATPs), multidrug-resistant protein-2 (MRP2) and MRP3. The function of OATPs and MRP2, which normally put drugs into bile are decreased, and MRP3 is increased so that substrates end up going toward the blood and then urine. Other drugs that are not as selective (e.g., acetaminophen) use these same transporters but also use other transporters that are not changed in NASH, or actually transporters that are increased in NASH. Additional data that shows that one of those other transporters (BCRP) is elevated in NASH so that it pushes drugs into bile. Drugs that use these other transporters that are not altered in NASH have a much smaller change in the amount that goes toward bile, suggesting that acetaminophen-glucuronide was only a relatively good diagnostic.

Determining if the accumulation of EZE-Gluc in plasma can be used as a metabolomic biomarker for NASH would be an important step in identifying patients who are at the greatest risk of developing cirrhosis, hepatocellular carcinoma, or possible adverse drug reactions. Although serum biomarkers can be measured to assess liver health and function in patients including serum bilirubin and aminotransferases, as previously mentioned, it has been observed in the clinic that significant liver damage (i.e. hepatic inflammation and fibrosis) and perhaps functional impairment are often not reflected by the results of serum chemistry screens of NAFLD patients. Specifically, blood panel tests performed on 106 patients with NAFLD here at the Banner University Medical Center (Tucson, AZ) failed to identify any abnormalities in liver enzyme levels in 15 individuals. However, upon further evaluation of these 15 patients with normal liver enzymes, 6 exhibited significant hepatic fibrosis. This finding is essential to our invention because it highlights a potentially critical weakness in the current clinical approach to diagnosing liver disease and strongly reinforces the clinical relevance of utilizing an exogenous metabolomic biomarker such as EZE-Gluc, as a superior, non-invasive, and safe means to assess liver health and metabolic function. Currently, the use of needle biopsy is the only conclusive means of fatty liver disease diagnosis and staging.

Additional advantages and aspects of the present invention are apparent in the following detailed description and claims.

### BRIEF DESCRIPTION OF THE SEVERAL VIEWS OF THE DRAWING(S)

The features and advantages of the present invention will become apparent from a consideration of the following detailed description presented in connection with the accompanying drawings in which:

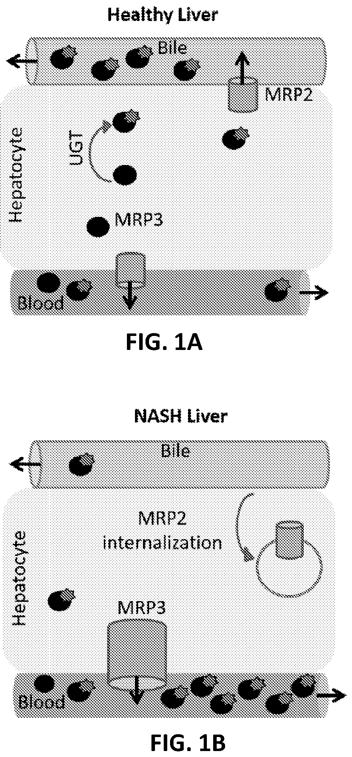

FIG. 1A shows the drug elimination pathway of a healthy liver, where a drug such as EZE is glucuronidated by UDP-glucuronosyltransferase (UGT) in enterocyte and hepatocytes.
FIG. 1B shows the drug elimination pathway of a non-alcoholic steatohepatitis (NASH) liver, where a shift in the preferential elimination pathway away from bile towards the blood is seen when EZE is given to a NASH patient, which results in a dramatically reduced biliary eliminated and increased plasma concentration of the glucuronidated EZE.

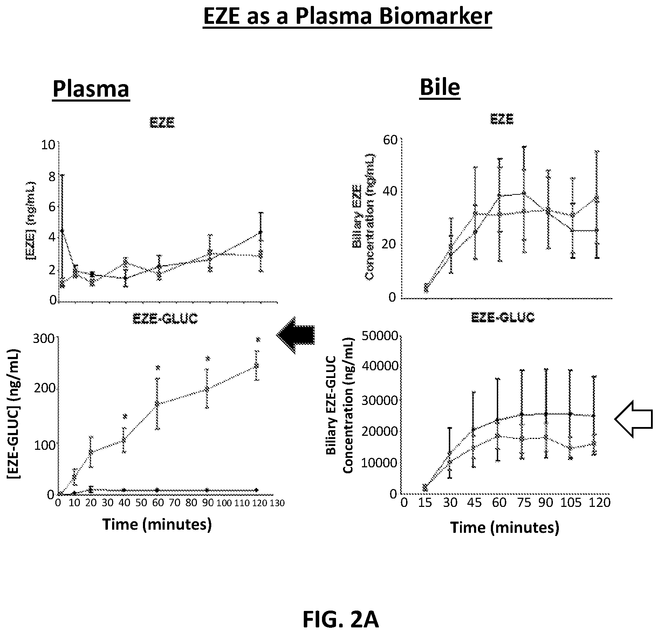

FIG. 2A shows the amount of ezetimibe (EZE) and glucuronidated ezetimibe (EZE-Gluc) in plasma and bile after oral administration of EZE to control and methionine- and choline-deficient diet fed mammalian model (MCD) rats. Blood and bile were collected and EZE and EZE-Gluc were measured by LC-MS/MS. The solid and open arrows indicate where NASH caused an increase and decrease, respectively, in the amount of compound in the biological fluid indicated.

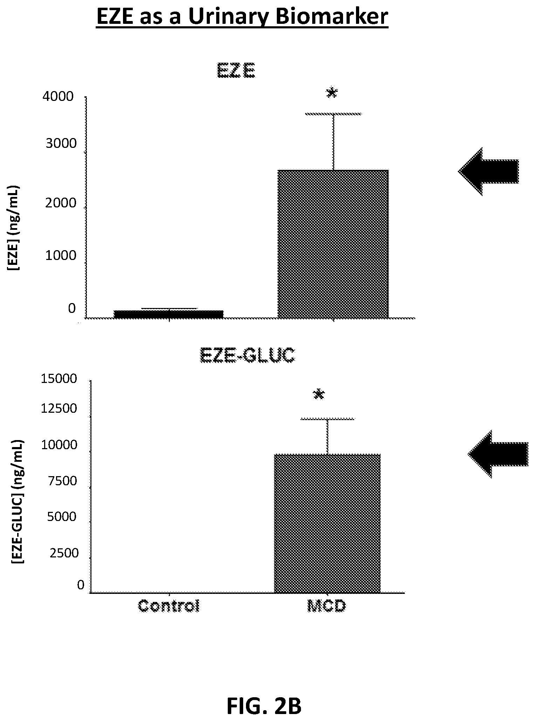

FIG. 2B shows the amount of ezetimibe (EZE) and glucuronidated ezetimibe (EZE-Gluc) in urine after intravenous administration of EZE to control and MCD (NASH) rats. Blood and bile were collected and EZE and ZE-Guc were measured by LC-MS/MS. The solid arrows indicate where NASH caused an increase in the amount of compound in the biological fluid indicated.

### DETAILED DESCRIPTION OF THE INVENTION

Referring now to FIGs. 1 - 2, the present invention features *in vitro* methods for the diagnosis and monitoring of a hepatic disorder using the metabolic biomarker, ezetimibe (EZE) and glucuronidated-ezetimibe (EZE-Gluc).

Referring now to FIG. 1A, one embodiment of a drug elimination pathway of a healthy liver is shown, where a drug such as EZE is glucuronidated by UDP-glucuronosyltransferase (UGT) in enterocyte and hepatocytes.

Referring now to FIG. 1B, one embodiment of drug elimination pathway of a NASH liver is shown. A shift in the preferential elimination pathway away from bile towards the blood is seen when EZE is given to a NASH patient. Without wishing to limit the present invention, MRP transporters MRP2 and MRP3 may alter human transporter function in livers of NASH versus healthy patients.

In an aspect, the present invention provides an *in vitro* method for diagnosing a hepatic disorder. The method comprises (a) determining an amount of EZE and/or EZE-Gluc in a plasma sample and/or a urine sample that was obtained from a human subject some time after the human subject has been administered EZE, and (b) comparing the amount of EZE and/or EZE-Gluc in one or both of the plasma sample and/or the urine sample to a control, and (c) identifying the human subject as having a hepatic disorder if the amount of EZE and/or EZE-Gluc in the plasma sample and/or urine sample is elevated relative to the control. The control or reference value is obtained from healthy individuals without a hepatic disorder, without signs/symptoms of a hepatic disorder, or without a risk for a hepatic disorder. In some embodiments, the subject can be identified as having a hepatic disorder if the amount of EZE and/or EZE-Gluc in one or both of the plasma sample and the urine sample is elevated relative to the control (e.g., compare plasma EZE-Gluc levels in human subject to plasma EZE-Gluc levels in control). In some embodiments, if the subject is below the reference threshold, the subject may not have hepatic disorder. In some embodiments, if the subject approaches the reference value, then the subject is at risk of developing the hepatic disorder. In some embodiments, if the subject surpasses the reference value, then the patient has the hepatic disorder. In some embodiments, if the subject surpasses a higher reference value, then the subject can be classified as having a more severe form of the hepatic disorder. In some embodiments, the amount of elevated EZE and/or EZE-Gluc levels is at least 50%, at least 100%, at least 200%, at least 300%, or at least 400% of control. In other embodiments, the amount of elevated EZE and/or EZE-Gluc is at least 10 times, 100 times, 1000 times, 10,000 times 100,000 times that of the control.

In some embodiments, the human subject may be at risk of, or diagnosed with, a hepatic disorder such as hepatitis, NAFLD, or NASH. Non-limiting examples of hepatic disorder co-morbidities may include metabolic syndrome, obesity, dyslipidemia, insulin resistance, hepatic fibrosis, and diabetes.

In some embodiments, the human subject may suffer from, or had previously suffered from, one or more conditions including, but not limited to, antitrypsin deficiency, Wilson's disease, fructosemia, galactosemia, Type III, IV, VI, IX, and X glycogen storage diseases, hemochromatosis, Gaucher's disease, Zellweger syndrome, tyrosinemia, bacterial infection, viral infection, parasitic infection, Budd-Chiari syndrome, alcoholism, cirrhosis, drug addiction, heart failure, hepatic veno-occlusive disease, portal vein thrombosis, fungal infection, protozoan infection, helminth infection, spirochete infection, sarcoidosis, ulcerative colitis, and Crohn's disease. In other embodiments, the human subject is a bariatric surgery patient. In still other embodiments, the human subject suffers from, or previously suffered from a disorder such as alcoholism or drug addiction.

In some embodiments, the amount of EZE and/or EZE-Gluc is measured in a plasma sample. The plasma sample for measuring the amount of EZE and/or EZE-Gluc may be taken from the subject after a period of time from administration of EZE. For example, the plasma sample may be taken from the subject about 1-10 minutes after EZE administration. In other embodiments, the plasma sample may be taken about 10-30 minutes, about 30-60 minutes, about 60-90 minutes, or about 90-120 minutes. In still other embodiments, the plasma sample may be taken about 2-4 hours, about 3-5 hours, or about 4-6 hours after EZE administration.

In other embodiments, the amount of EZE and/or EZE-Gluc is measured in a urine sample. The urine sample for measuring the amount of EZE and/or EZE-Gluc may be taken from the subject after a period of time from administration of EZE. For instance, the urine sample may be taken from the subject about 60-360 minutes after EZE administration. In some embodiments, the urine sample may be taken about 1-2 hours, about 2-4 hours, about 3-5, or about 4-6 hours after EZE administration.

It is to be understood that the present invention is not limited to the aforementioned times. In preferred embodiments, the amount of time between administering EZE to the subject and taking the plasma and/or urine sample is sufficient to allow for EZE and/or EZE-Gluc to be detected and levels thereof measured by any appropriate system, e.g., LC, MS, LC/MS, immune assays.

In some embodiments, the subject may be administered about 0.001 to 10 mg of EZE per dose. In other embodiments, the subject may be administered about 0.001 to 0.01 mg, about 0.01 to 0.1 mg, about 1.0 to 3.0 mg, about 3.0 to 5.0 mg, about 5.0 to 7.0 mg, about 7.0 to 9.0 mg, or about 9.0 to 11.0 mg EZE per dose. In some preferred embodiments, the subject may be administered about 1 mg EZE per dose. In other preferred embodiments, the subject may be administered no more than about 10 mg EZE per dose. In a preferred embodiment, EZE may be administered to subject at least once. In another preferred embodiment, EZE may be administered to subject once or twice. In still other embodiments, EZE may be administered to subject for any number of times to obtain a reliable sample size.

It is to be understood that the present invention is not limited to the aforementioned doses. In preferred embodiments, the amount of EZE administered to the subject may be a sufficiently low amount such that EZE has minimal to no therapeutic effect, yet still allowing EZE and/or EZE-Gluc to be detectable by any appropriate system (e.g., LC, MS, LC/MS, immune assays) to indicate disease. In some preferred embodiments, the maximum amount of EZE administered to the subject may be an amount that does not have any toxic effects. In other preferred embodiments, the maximum amount of EZE administered to the subject may be a therapeutic dose. In yet other preferred embodiments, the amount of EZE administered to the subject may be any dose of EZE that allows for measurement of EZE-Gluc levels.

According to other embodiments, the present invention provides an *in vitro* method of monitoring progression of a hepatic disorder or disease in a subject over time. In one embodiment, the method comprises measuring an amount of EZE and/or EZE-Gluc in a fluid sample (e.g., plasma and/or urine) that was obtained from the subject after a period of time has passed after the subject has been administered EZE, and comparing the amount of EZE and/or EZE-Gluc to a reference value in healthy patients without the disorder and without signs or symptoms of the disorder. In preferred embodiments, the plasma EZE-Gluc level in the subject is compared to the plasma EZE-Gluc level in the reference, the plasma EZE level in the subject is compared to the plasma EZE level in the reference, the urine EZE-Gluc level in the subject is compared to the urine EZE-Gluc level in the reference, and/or the urine EZE-Gluc level in the subject is compared to the urine EZE-Gluc level in the reference. In one embodiment, a deviation of the amount of EZE-Gluc from the reference value indicates a stage of the hepatic disease. In some embodiments, if the subject is below the reference threshold, the subject may not have NASH. In some embodiments, if the subject approaches the reference value, then they may be at risk for NASH. In some embodiments, if the subject surpasses the reference value, then the patient has NASH. In some embodiments, if the subject surpasses a higher reference value, then the patient has a more severe form of NASH As a non-limiting example, the reference threshold may be about 10 ng/ml of plasma depending on the dose, fluid being analysed, and time from administration. In some embodiments, the deviation in the amount of EZE and/or EZE-Gluc may be at least 50%, at least 100%, at least 200%, at least 300%, or at least 400% of the reference. In other embodiments, the amount of elevated EZE and/or EZE-Gluc is at least 10 times, 100 times, 1000 times, 10,000 times, or at least 100,000 times that of the reference. In some embodiments, the monitoring occurs at select times throughout the disorder, treatment, or patient care management comprising intervals of daily, weekly, monthly, and/or yearly.

In an aspect, the present invention provides an *in vitro* method of monitoring progression of a hepatic disorder or disease in a human subject. The method comprises (a) measuring an amount of EZE and/or EZE-Gluc in a non-biopsy, fluid sample that was obtained from the human subject some time after the human subject has been administered EZE, and (b) comparing the amount of EZE and/or EZE-Gluc to a reference value in healthy patients without the disorder and without signs or symptoms of the disorder, wherein a deviation of the amount of EZE and/or EZE/Gluc from the reference value indicates a stage of the hepatic disease. In preferred embodiments, the plasma EZE-Gluc level in the subject is compared to the plasma EZE-Gluc level in the reference, the plasma EZE level in the subject is compared to the plasma EZE level in the reference, the urine level of EZE-Gluc level in the subject is compared to the urine EZE-Gluc level in the reference, and/or the urine EZE level in the subject is compared to the urine EZE level in the reference. Without wishing to limit the invention to a particular theory or mechanism, a deviation of the amount of EZE-Gluc from the reference value may be indicative of a hepatic disorder and the stage of the hepatic disease. In some embodiments, the amount of deviated EZE and/or EZE-Gluc levels is at least 50%, at least 100%, at least 200%, at least 300%, or at least 400% of the reference. Non-limiting examples comprise the plasma EZE-Gluc level in the subject being 200% higher compared to the plasma level of EZE-Gluc in the reference or the urine EZE-Gluc level in the subject being 100% higher compared to the urine level of EZE-Gluc in the reference. In other embodiments, the amount of elevated EZE and/or EZE-Gluc is at least 10 times, 100 times, 1000 times, 10,000 times, or at least 100,000 times that of the reference. A non-limiting example comprises the plasma EZE-Gluc level in the subject is 1000 times higher compared to the plasma level of EZE-Gluc in the reference.

In preferred embodiments, the method further comprises prescribing a therapy to the patient stage of the hepatic disease. In some embodiments, prescribing a therapy to the patient may involve one or more of dietary changes, exercise regimens, lifestyle changes, surgical interventions, and use of medications.

In an aspect, the present invention features an *in vitro* method of monitoring the effectiveness of a treatment for a hepatic disorder or disease in a human subject in need of such treatment, wherein the human subject has been administered ezetimibe (EZE) prior to initiation of the treatment for the hepatic disorder and at any time following the initiation of treatment for the hepatic disorder, wherein prior to treatment, initiation administration of EZE serves as an initial, baseline assessment. The method comprises (a) measuring an amount of EZE and/or ezetimibe-glucuronide (EZE-Gluc) in a non-biopsy, fluid sample that was obtained from the human subject some time after the human subject has been administered EZE, (b) comparing the amount of EZE and/or EZE-Gluc between the initial (pre-treatment), baseline and post-treatment initiation (during treatment) values and/or to a reference value (e.g., compare plasma EZE-Gluc level in subject being monitored to the plasma EZE-Gluc level in reference) where a deviation of the amount of EZE and/or EZE-Gluc from the reference and/or baseline value indicates a stage of the hepatic disease, and (c) monitoring the effectiveness of a therapy to the subject based on the stage of hepatic disorder defined in (b), wherein if the amount of EZE and/or EZE-Gluc in the biological fluid sample has decreased over time relative to the initial, baseline assessment prior to initiation of treatment, then the treatment is efficacious. The change of levels of EZE and/or EZE-Gluc from prior to initiation of therapy and between the subject and the reference indicates the efficacy of the treatment. Without wishing to limit the invention to a particular theory or mechanism, a deviation of the amount of EZE and/or EZE-Gluc from the reference value indicates a stage of the hepatic disease.

In preferred embodiments, the method comprises monitoring a treatment that is prescribed to the patient stage of the hepatic disease. In some embodiments, the subject can be identified as still having the hepatic disorder if the amount of EZE and/or EZE-Gluc in one or both of the plasma sample and the urine sample is elevated relative to the reference and/or to the baseline value (e.g., compare plasma EZE-Gluc levels in human subject to plasma EZE-Gluc levels in reference and/or to baseline levels prior to initiation of therapy; compare urine EZE-Gluc levels in human subject to urine EZE-Gluc levels in reference and/or baseline), possibly requiring a change in treatment. In some embodiments, if the subject has a level that is below the level obtained prior to initiating the treatment, the subject may be receiving benefit from the treatment. In some embodiments, if the level in a subject decreases and approaches the reference value, then the subject may be considered cured. In some embodiments, if the subject has a level that surpasses the initial value, then the patient continues to have the hepatic disorder. In some embodiments, the amount of elevated or deviated EZE and/or EZE-Gluc levels is at least 50%, at least 100%, at least 200%, at least 300%, or at least 400% of control (e.g., compare plasma EZE-Gluc in subject to plasma level of EZE-Gluc in the control). In other embodiments, the amount of elevated EZE and/or EZE-Gluc is at least 10 times, 100 times, 1000 times, 10,000 times 100,000 times that of the control (e.g., compare plasma EZE-Gluc in subject to plasma level of EZE-Gluc in the control). In other embodiments, the amount of elevated EZE and/or EZE-Gluc is compared to the patient's EZE and/or EZE-Gluc levels prior to initiation of therapy where any decrease (e.g., at least 1%) toward reference value would indicate improvement, abatement, or resolution of the hepatic disorder. In some embodiments, the decrease indicative of treatment efficacy is at least 1%, at least 5%, at least 10%, at least 25%, at least 50%, at 75%, or at least 100%.

In preferred embodiments, the time of EZE administration for biomarker testing prior to initiation of a treatment for a hepatic disorder comprises up to 0 day (e.g., same day as treatment initiation), up to 1 day, up to 2 days, up to 3 days, or up to 6 months prior to treatment initiation. In some embodiments, the time of EZE administration for biomarker testing prior to initiation of a treatment for a hepatic disorder may be any number of days appropriate to allow for the EZE measurement to be used in accordance with the present invention. In some embodiments, the treatment to the patient may change based on monitoring results and may involve one or more of dietary changes, exercise regimens, lifestyle changes, surgical interventions, and use of medications.

In an aspect, the present invention further features an *in vitro* method for delineating non-alcoholic steatohepatitis (NASH) from simple steatosis in a human subject. The method comprises (a) determining an amount of EZE and/or ezetimibe-glucuronide (EZE-Gluc) in a plasma sample and/or a urine sample that was obtained from the human subject after some time the human subject has been administered ezetimibe (EZE), (b) comparing the amount of EZE and/or EZE-Gluc in one or both of the plasma sample and the urine sample to a reference value, wherein the reference is from subjects with steatosis, and (c) identifying the human subject as having NASH if the amount of EZE and/or EZE-Gluc in one or both of the plasma sample and the urine sample is elevated relative to the steatosis reference. In some embodiments, the amount of elevated or deviated EZE and/or EZE-Gluc levels is at least 50%, at least 100%, at least 200%, at least 300%, or at least 400% of steatosis (e.g., compare plasma EZE-Gluc in subject to plasma level of EZE-Gluc in the steatosis reference). In other embodiments, the amount of elevated EZE and/or EZE-Gluc is at least 10 times, 100 times, 1000 times, 10,000 times 100,000 times that of the steatosis (e.g., compare plasma EZE-Gluc in subject to plasma level of EZE-Gluc in the steatosis reference).

The present invention also features a method for determining the efficacy of a therapeutic treatment for NASH in patients in need of such therapy. In some embodiments, the method comprises determining an amount of EZE and/or EZE-Gluc in a plasma sample and/or a urine sample that was obtained from the human subject after some time the human subject has been administered EZE prior to initiation of treatment for NASH (to serve as initial or baseline assessment) and at any time following initiation of NASH treatment, comparing the amount of EZE and/or EZE-Gluc in one or both of the plasma sample and the urine sample to the initial EZE and/or EZE-Gluc in one or both of the plasma sample and the urine sample of the patient prior to initiation of therapy, and determining the efficacy of the treatment if the amount of EZE and/or EZE-Gluc in one or both of the plasma sample and the urine sample has decreased over time relative to the initial test prior to initiation of treatment, wherein any decrease over time (e.g., at least 1%) indicates that the treatment is efficacious. In some embodiments, the decrease indicative of treatment efficacy is at least 1%, at least 5%, at least 10%, at least 25%, at least 50%, at 75%, or at least 100%.

In some embodiments, the time of EZE administration for biomarker testing prior to initiation of a treatment for a hepatic disorder comprises up to 0 day (e.g., same day as treatment initiation), up to 1 day, up to 2 days, up to 3 days, or up to 6 months prior to treatment. In some embodiments, the time of EZE administration for biomarker testing prior to initiation of a treatment for a hepatic disorder may be any number of days appropriate to allow for the EZE measurement to be used in accordance with the present invention. In some embodiments, the treatment to the patient may change based on monitoring results and may involve one or more of dietary changes, exercise regimens, lifestyle changes, surgical interventions, and use of medications.

Consistent with previous embodiments, other markers may be used that can distinguish NAFL from NASH, alternatively or in conjunction with EZE-Gluc. Non-limiting examples of these other biomarkers include CK-18 as well as perillipin-1 (PLIN-1) and PLIN-2 lipid droplet proteins, age, weight, ALT, AST, triglyceride, diabetes, TIMP-1, hepascore, hyaluronic acid, elastography, acoustic radiation, fibroscan, MRI, CT, or PET scans.

### EXAMPLES

The following is a non-limiting example of the present invention. It is to be understood that said example is not intended to limit the present invention in any way.

### Example 1: EZE-GLUC Increased Plasma Retention and Decreased Biliary Elimination in a NASH Animal Model

Ezetimibe was orally administered to control and MCD rats. Blood and bile were collected and EZE and ZE-Guc were measured by LC-MS/MS. FIG. 2A shows the amount of ezetimibe (EZE) and glucuronidated ezetimibe (EZE-Gluc) in plasma and bile after the oral administration of EZE to control and MCD (NASH) rats. The solid and open arrows indicate where NASH caused an increase and decrease, respectively, in the amount of compound in the biological fluid indicated.

Ezetimibe was intravenously administered to control and MCD rats. Blood and bile were collected and EZE and EZE-Guc were measured by LC-MS/MS. FIG. 2B shows the amount of ezetimibe (EZE) and glucuronidated ezetimibe (EZE-Gluc) in urine after the intravenous administration of EZE to control and MCD (NASH) rats. The solid arrows indicate where NASH caused an increase in the amount of compound in the biological fluid indicated.

### Example 2: Method of EZE/EZE-Gluc Assessment in Human Subjects

A subject at risk for a hepatic disorder arrives at a testing site on the morning of the assessment after an overnight fast who was previously advised to avoid any product containing either APAP or EZE (including TYLENOL^{®}, NyQuil^{®}, Robitussin^{®}, Zetia etc.) for at least three days prior to the assessment. Following baseline blood (20 cc) and urine collection, the subject receives one standard oral dose of EZE (e.g., 10 mg). Subjects are allowed access to food and water during the 6 h assessment. Blood is collected at 0.25, 0.5, 1, 1.5, 2, 3, 4, and 6 h post drug administration by an appropriately trained professional (e.g., attending nursing staff). Approximately 7 cc of blood is collected into a heparinized vacutainer tube at each time point, and the samples are centrifuged within 30 min of blood collection. Due to the effects of patient hydration and kidney health on the variability in urine concentration, urine levels of metabolites are calculated as a total amount rather than as a concentration. Urine is collected at 1, 2, 4 and 6 h, the total volume is noted, and a small (10 mL) sample is reserved for LC-MS/MS analysis.

In some embodiments, biochemical analysis is performed on pre-dose blood samples, and measure alkaline phosphatase (AP), aspartate aminotransferase (AST), alanine aminotransferase (ALT), gamma-glutamyl transferase (γGT), total cholesterol and fractions, triglycerides, fasting plasma glucose, and insulin. Height and weight measurements will also be recorded for BMI calculation and statistical analysis. Cytokeratin-18 levels will be determined using a commercially available ELISA kit.

Using the collected blood and urine samples, ezetimibe and metabolite quantification can be performed according to standard procedures. Briefly, tolbutamide is used as an internal standard, and samples are extracted using Oasis^{®} HLB solid phase extraction. EZE and EZE-Gluc are measured according to standard analytical procedures (20,58). For example, the LC-MS/MS system is an Acquity Ultra Performance Liquid Chromatography (UPLC^{®}) system with an auto-sampler (Waters, Milford, MA) coupled with a Waters Micromass Quattro Premier^{™} XE tandem mass spectrometer (Waters) equipped with MassLynx^{™} 4.1 software (Waters). The chromatography consists of a gradient beginning at 20:80 (v/v) acetonitrile-water and ending at 80:20 (v/v) acetonitrile-water for 7 min with a flow rate of 0.25 mL/min on an Acquity UPLC^{®} BEH C18 column (1.7 µm; 2.1 × 50 mm; Waters). Electrospray ionization in the negative ion mode is used, and the analytes are detected using a multiple reaction monitoring (MRM) method. The precursor and product ions (*m*/*z*) are 408 and 271, respectively, for EZE, 584 and 271, respectively, for EZE-Gluc, and 269 to 170, respectively, for the internal standard.

The levels of EZE and/or EZE-Gluc are then analyzed and compared (e.g., EZE to EZE levels; EZE-Gluc to EZE-Gluc levels) to a control reference value obtained from healthy individuals without a hepatic disorder, without signs/symptoms of a hepatic disorder, or without a risk for a hepatic disorder. For example, average peak blood concentrations of 92 ng/mL and 4.5 ng/mL for EZE-Gluc and EZE, respectively, were found in normal healthy individuals after a 10 mg oral dose of EZE. In some embodiments, the subjects with NASH or at risk of NASH have levels of EZE-Gluc at least 50%, at least 100%, at least 200%, at least 300%, or at least 400% higher than the control at early time points, for example one hour in blood or two hours in urine. At later timepoints (e.g., after 2 hours in the blood; 4 hours in the urine), the levels become much more elevated, as much as 10-100,000 times as compared to the control. In some embodiments, the elevation of EZE-Gluc will only be present at the stage of NASH. In other embodiments, EZE-Gluc can be used to differentiate between grades of NASH. In certain embodiments, the levels of EZE-Gluc in subjects with steatosis will be no different than control.

As used herein, the term "about" refers to plus or minus 10% of the referenced number. Various modifications of the invention, in addition to those described herein, will be apparent to those skilled in the art from the foregoing description.

The scope of the invention is only to be limited by the following claims. In some embodiments, the figures presented in this patent application are drawn to scale, including the angles, ratios of dimensions, etc. In some embodiments, the figures are representative only and the claims are not limited by the dimensions of the figures. In some embodiments, descriptions of the inventions described herein using the phrase "comprising" includes embodiments that could be described as "consisting essentially of" or "consisting of", and as such the written description requirement for claiming one or more embodiments of the present invention using the phrase "consisting essentially of" or "consisting of" is met.

## Claims

1. An *in vitro* method of diagnosing a hepatic disorder in a human subject at risk of the hepatic disorder, said method comprising:
a) determining an amount of ezetimibe (EZE) and/or ezetimibe-glucuronide (EZE-Gluc) in a plasma sample and/or urine sample that was obtained from the human subject some time after the human subject has been administered ezetimibe (EZE);
b) comparing the amount of EZE and/or EZE-Gluc in the plasma sample and/or urine sample to a control; and
c) identifying the human subject as having a hepatic disorder if the amount of EZE and/or EZE-Gluc in the plasma sample and/or urine sample is elevated relative to the control.

2. An *in vitro* method of monitoring progression of a hepatic disorder in a human subject, said method comprising:
a) measuring an amount of ezetimibe (EZE) and/or ezetimibe-glucuronide (EZE-Gluc) in a non-biopsy, fluid sample that was obtained from the human subject some time after the human subject has been administered ezetimibe (EZE); and
b) comparing the amount of EZE and/or EZE-Gluc to a reference value, wherein a deviation of the amount of EZE and/or EZE-Gluc from the reference value indicates a stage of the hepatic disease.

3. An *in vitro* method of monitoring effectiveness of a treatment for a hepatic disorder in a human subject, wherein the human subject has been administered ezetimibe (EZE) prior to initiation of the treatment for the hepatic disorder and at any time following the initiation of treatment for the hepatic disorder, wherein prior to treatment, initiation administration of EZE serves as an initial, baseline assessment, wherein the method comprises:
a) measuring an amount of EZE and/or ezetimibe-glucuronide (EZE-Gluc) in a non-biopsy, fluid sample that was obtained from the human subject some time after the human subject has been administered ezetimibe (EZE);
b) comparing the amount of EZE and/or EZE-Gluc between the initial, baseline and post-treatment initiation and/or to a reference value, where a deviation of the amount of EZE and/or EZE-Gluc from the reference and/or baseline value indicates a stage of the hepatic disease; and
c) monitoring the effectiveness of a therapy to the subject based on the stage of the hepatic disorder defined in (b), wherein if the amount of EZE and/or EZE-Gluc in the biological fluid sample has decreased over time relative to the initial, baseline assessment prior to initiation of treatment, then the treatment is efficacious.

4. An *in vitro* method of delineating non-alcoholic steatohepatitis (NASH) from simple steatosis in a human subject, said method comprising:
a) determining an amount of ezetimibe (EZE) and/or ezetimibe-glucuronide (EZE-Gluc) in a plasma sample and/or a urine sample obtained from the human subject some time after the human subject has been administered EZE;
b) comparing the amount of EZE and/or EZE-Gluc in one or both of the plasma sample and the urine sample to a reference, wherein the reference is from subjects with steatosis; and
c) identifying the human subject as having NASH if the amount of EZE and/or EZE-Gluc in one or both of the plasma sample and the urine sample is elevated relative to the control.

5. The method according to any one of claims 1-3, wherein the human subject at risk of or with a hepatic disorder suffers from one or more of a metabolic syndrome, obesity, dyslipidemia, insulin resistance, or diabetes.

6. The method according to any one of claims 1-3, wherein the human subject is at risk of non-alcoholic steatohepatitis (NASH), hepatic fibrosis, or hepatitis.

7. The method of claim 6, wherein the human subject at risk of NASH suffers from one or more of non-alcoholic fatty liver disease (NAFL), metabolic syndrome, obesity, dyslipidemia, insulin resistance, or diabetes.

8. The method of claim 6, wherein the human subject at risk of hepatic fibrosis suffers from, or previously suffered from, one or more conditions selected from the group consisting of or antitrypsin deficiency, Wilson's disease, fructosemia, galactosemia, Type III, IV, VI, IX, and X glycogen storage diseases, hemochromatosis, Gaucher's disease, Zellweger syndrome, tyrosinemia, bacterial infection, viral infection, parasitic infection, Budd-Chiari syndrome, alcoholism, drug addiction, heart failure, hepatic veno-occlusive disease, or portal vein thrombosis.

9. The method of claim 6, wherein the human subject at risk of hepatitis suffers from, or previously suffered from, one or more disorders selected from the group consisting of alcoholism, drug addiction, bacterial infection, viral infection, fungal infection, protozoan infection, helminth infection, spirochete infection, sarcoidosis, ulcerative colitis, and Crohn's disease.

10. The method according to any one of claims 1-3, wherein the human subject is an obese human and/or a bariatric surgery patient.

11. The method according to claim 1 or 2, wherein the control or reference value is from an aggregate population of asymptomatic individuals without the hepatic disorder, without risk of hepatic disorder, and/or without signs or symptoms of a hepatic disorder.

12. The method according to any one of claims 1-2, wherein the amount of EZE and/or EZE-Gluc is elevated by at least 50% relative to the control or reference value.

## Patentansprüche

1. In-vitro-Verfahren zum Diagnostizieren einer Lebererkrankung bei einem menschlichen Subjekt, bei dem ein Risiko für die Lebererkrankung besteht, das Verfahren umfassend:
a) Bestimmen einer Menge an Ezetimib (EZE) und/oder Ezetimib-Glucuronid (EZE-Gluc) in einer Plasmaprobe und/oder Urinprobe, die von dem menschlichen Subjekt einige Zeit nach Verabreichen von Ezetimib (EZE) erlangt wird;
b) Vergleichen der Menge an EZE und/oder EZE-Gluc in der Plasmaprobe und/oder Urinprobe mit einer Kontrolle; und
c) Identifizieren des menschlichen Subjekts als eines, das eine Lebererkrankung hat, wenn die Menge an EZE und/oder EZE-Gluc in der Plasmaprobe und/oder Urinprobe in Bezug auf die Kontrolle erhöht ist.

2. In-vitro-Verfahren zum Überwachen eines Fortschreitens einer Lebererkrankung bei einem menschlichen Subjekt, das Verfahren umfassend:
a) Messen einer Menge an Ezetimib (EZE) und/oder Ezetimib-Glucuronid (EZE-Gluc) in einer nicht-biopsischen, fluiden Probe, die von dem menschlichen Subjekt einige Zeit nach Verabreichen von Ezetimib (EZE) an das menschliche Subjekt erlangt wird; und
b) Vergleichen der Menge von EZE und/oder EZE-Gluc mit einem Referenzwert, wobei eine Abweichung der Menge von EZE und/oder EZE-Gluc von dem Referenzwert ein Stadium der Leberkrankheit angibt.

3. In-vitro-Verfahren zum Überwachen der Wirksamkeit einer Behandlung einer Lebererkrankung bei einem menschlichen Subjekt, wobei dem menschlichen Subjekt vor Beginn der Behandlung der Lebererkrankung und zu jedem Zeitpunkt nach Beginn der Behandlung der Lebererkrankung Ezetimib (EZE) verabreicht wurde, wobei vor einer Behandlung eine Einleitung einer Verabreichung von EZE als anfängliche Ausgangsbewertung dient, wobei das Verfahren Folgendes umfasst:
a) Messen einer Menge an EZE und/oder Ezetimib-Glucuronid (EZE-Gluc) in einer nicht-biopsischen, fluiden Probe, die von dem menschlichen Subjekt einige Zeit nach Verabreichen von Ezetimib (EZE) an das menschliche Subjekt erlangt wird;
b) Vergleichen der Menge an EZE und/oder EZE-Gluc zwischen dem Anfangs-, Ausgangs- und Nachbehandlungsbeginn und/oder einem Referenzwert, wobei eine Abweichung der Menge an EZE und/oder EZE-Gluc von dem Referenz- und/oder Ausgangswert ein Stadium der Leberkrankheit angibt; und
c) Überwachen der Wirksamkeit einer Therapie für den Patienten basierend auf dem in (b) definierten Stadium der Lebererkrankung, wobei, wenn die Menge an EZE und/oder EZE-Gluc in der biologischen fluiden Probe im Lauf der Zeit in Bezug auf die anfängliche Ausgangsbewertung vor Beginn der Behandlung abgenommen hat, die Behandlung wirksam ist.

4. In-vitro-Verfahren zum Abgrenzen von nicht-alkoholischer Steatohepatitis (NASH) von einfacher Steatose bei einem menschlichen Subjekt, das Verfahren umfassend:
a) Bestimmen einer Menge an Ezetimib (EZE) und/oder Ezetimib-Glucuronid (EZE-Gluc) in einer Plasmaprobe und/oder einer Urinprobe, erlangt von dem menschlichen Subjekt einige Zeit nach Verabreichen von EZE;
b) Vergleichen der Menge an EZE und/oder EZE-Gluc in einer oder beiden von der Plasmaprobe und der Urinprobe mit einer Referenz, wobei die Referenz von Subjekten mit Steatose stammt; und
c) Identifizieren, dass das menschliche Subjekt NASH hat, wenn die Menge an EZE und/oder EZE-Gluc in einer oder beiden von der Plasmaprobe und der Urinprobe in Bezug auf die Kontrolle erhöht ist.

5. Verfahren nach einem der Ansprüche 1 bis 3, wobei das menschliche Subjekt, bei dem ein Risiko für eine Lebererkrankung besteht oder das eine solche hat, an einem oder mehreren von einem metabolischen Syndrom, Fettleibigkeit, Dyslipidämie, Insulinresistenz oder Diabetes leidet.

6. Verfahren nach einem der Ansprüche 1-3, wobei bei dem menschlichen Subjekt ein Risiko für nicht-alkoholische Steatohepatitis (NASH), Leberfibrose oder Hepatitis besteht.

7. Verfahren nach Anspruch 6, wobei das menschliche Subjekt, bei ein Risiko für NASH besteht, an einer oder mehreren von nicht-alkoholischer Fettlebererkrankung (NAFL), metabolischem Syndrom, Fettleibigkeit, Dyslipidämie, Insulinresistenz oder Diabetes leidet.

8. Verfahren nach Anspruch 6, wobei das menschliche Subjekt, bei ein Risiko für Leberfibrose besteht, an einem oder mehreren Zuständen leidet, oder zuvor gelitten hat, die ausgewählt sind, aus der Gruppe, bestehend aus Antitrypsinmangel, Wilson-Krankheit, Fruktosämie, Galaktosämie, Glykogenspeicherkrankheiten vom Typ III, IV, VI, IX und X, Hämochromatose, Gaucher-Krankheit, Zellwegersyndrom, Tyrosinämie, bakterieller Infektion, Virusinfektion, parasitärer Infektion, Budd-Chiari-Syndrom, Alkoholismus, Drogenabhängigkeit, Herzinsuffizienz, Lebervenenverschlusskrankheit oder Pfortader-Thrombose.

9. Verfahren nach Anspruch 6, wobei das menschliche Subjekt, bei ein Risiko für Hepatitis besteht, an einer oder mehreren Erkrankungen leidet, oder zuvor darunter gelitten hat, die ausgewählt sind aus der Gruppe, bestehend aus Alkoholismus, Drogenabhängigkeit, bakterieller Infektion, Virusinfektion, Pilzinfektion, Protozoeninfektion, Helmintheninfektion, Spirochäteninfektion, Sarkoidose, Colitis ulcerosa und Morbus Crohn.

10. Verfahren nach einem der Ansprüche 1-3, wobei das menschliche Subjekt ein adipöser menschlicher und/oder ein bariatrischer Chirurgiepatient ist.

11. Verfahren nach Anspruch 1 oder 2, wobei der Kontroll- oder Referenzwert aus einer Gesamtpopulation asymptomatischer Personen ohne die Lebererkrankung, ohne das Risiko für eine Lebererkrankung und/oder ohne Anzeichen oder Symptome für eine Lebererkrankung stammt.

12. Verfahren nach einem der Ansprüche 1-2, wobei die Menge an EZE und/oder EZE-Gluc um mindestens 50 % in Bezug auf den Kontroll- oder Referenzwert erhöht ist.

## Revendications

1. Procédé *in vitro* de diagnostic d'un trouble hépatique chez un sujet humain à risque de présenter le trouble hépatique, ledit procédé comprenant :
a) la détermination d'une quantité d'ézétimibe (EZE) et/ou d'ézétimibe-glucuronide (EZE-Gluc) dans un échantillon de plasma et/ou un échantillon d'urine qui a été prélevé sur le sujet humain peu après l'administration d'ézétimibe (EZE) au sujet humain ;
b) la comparaison de la quantité d'EZE et/ou d'EZE-Gluc dans l'échantillon de plasma et/ou l'échantillon d'urine à un témoin ; et
c) l'identification du sujet humain comme présentant un trouble hépatique si la quantité d'EZE et/ou d'EZE-Gluc dans l'échantillon de plasma et/ou l'échantillon d'urine est élevée par rapport au témoin.

2. Procédé *in vitro* de surveillance de la progression d'un trouble hépatique chez un sujet humain, ledit procédé comprenant :
a) la mesure d'une quantité d'ézétimibe (EZE) et/ou d'ézétimibe-glucuronide (EZE-Gluc) dans un échantillon de fluide non biopsique qui a été prélevé sur le sujet humain peu après l'administration d'ézétimibe (EZE) au sujet humain ; et
b) la comparaison de la quantité d'EZE et/ou d'EZE-Gluc à une valeur de référence, dans lequel un écart de la quantité d'EZE et/ou d'EZE-Gluc par rapport à la valeur de référence indique un stade de la maladie hépatique.

3. Procédé *in vitro* de surveillance de l'efficacité d'un traitement d'un trouble hépatique chez un sujet humain, dans lequel de l'ézétimibe (EZE) a été administré au sujet humain avant le début du traitement du trouble hépatique et à tout moment après le début du traitement du trouble hépatique, dans lequel avant le traitement, l'administration initiale d'EZE sert d'évaluation initiale de base, dans lequel le procédé comprend :
a) la mesure d'une quantité d'EZE et/ou d'ézétimibe-glucuronide (EZE-Gluc) dans un échantillon de fluide non biopsique qui a été prélevé sur le sujet humain peu après l'administration d'ézétimibe (EZE) au sujet humain ;
b) la comparaison de la quantité d'EZE et/ou d'EZE-Gluc entre l'instauration de base initiale et post-traitement et/ou à une valeur de référence, un écart de la quantité d'EZE et/ou d'EZE-Gluc par rapport à la valeur de référence et/ou de base indiquant un stade de la maladie hépatique ; et
c) la surveillance de l'efficacité d'un traitement chez le sujet sur la base du stade du trouble hépatique défini en (b), dans lequel si la quantité d'EZE et/ou d'EZE-Gluc dans l'échantillon de fluide biologique a diminué au fil du temps par rapport à l'évaluation initiale de base avant l'instauration du traitement, alors le traitement est efficace.

4. Procédé *in vitro* de distinction de la stéatohépatite non alcoolique (NASH) d'une stéatose simple chez un sujet humain, ledit procédé comprenant :
a) la détermination d'une quantité d'ézétimibe (EZE) et/ou d'ézétimibe-glucuronide (EZE-Gluc) dans un échantillon de plasma et/ou un échantillon d'urine prélevé sur le sujet humain peu après l'administration d'ézétimibe (EZE) au sujet humain ;
b) la comparaison de la quantité d'EZE et/ou d'EZE-Gluc dans l'un et/ou l'autre de l'échantillon de plasma et de l'échantillon d'urine à une référence, dans lequel la référence est issue de sujets atteints de stéatose ; et
c) l'identification du sujet humain comme présentant une NASH si la quantité d'EZE et/ou d'EZE-Gluc dans l'un et/ou l'autre de l'échantillon de plasma et de l'échantillon d'urine est élevée par rapport au témoin.

5. Procédé selon l'une quelconque des revendications 1-3, dans lequel le sujet humain à risque ou atteint d'un trouble hépatique souffre d'un ou plusieurs élément parmi le syndrome métabolique, l'obésité, la dyslipidémie, la résistance à l'insuline ou le diabète.

6. Procédé selon l'une quelconque des revendications 1-3, dans lequel le sujet humain est à risque de présenter une stéatohépatite non alcoolique (NASH), une fibrose hépatique ou une hépatite.

7. Procédé selon la revendication 6, dans lequel le sujet humain à risque de présenter une NASH souffre d'un ou plusieurs éléments parmi la stéatose hépatique non alcoolique (NAFL), le syndrome métabolique, l'obésité, la dyslipidémie, la résistance à l'insuline ou le diabète.

8. Procédé selon la revendication 6, dans lequel le sujet humain à risque de présenter une fibrose hépatique souffre ou a déjà souffert d'une ou plusieurs affections choisies dans le groupe consistant en une carence en antitrypsine, la maladie de Wilson, la fructosémie, la galactosémie, les glycogénoses de type III, IV, VI, IX et X, l'hémochromatose, la maladie de Gaucher, le syndrome de Zellweger, la tyrosinémie, une infection bactérienne, une infection virale, une infection parasitaire, le syndrome de Budd-Chiari, l'alcoolisme, la toxicomanie, l'insuffisance cardiaque, la maladie veino-occlusive hépatique ou la thrombose de la veine porte.

9. Procédé selon la revendication 6, dans lequel le sujet humain à risque de présenter une hépatite souffre ou a déjà souffert d'un ou plusieurs troubles choisis dans le groupe consistant en l'alcoolisme, la toxicomanie, l'infection bactérienne, l'infection virale, l'infection fongique, l'infection à protozoaires, l'infection par les helminthes, l'infection aux spirochètes, la sarcoïdose, la colite ulcéreuse et la maladie de Crohn.

10. Procédé selon l'une quelconque des revendications 1-3, dans lequel le sujet humain est un humain obèse et/ou un patient ayant subi une chirurgie bariatrique.

11. Procédé selon la revendication 1 ou 2, dans lequel la valeur témoin ou de référence provient d'une population agrégée d'individus asymptomatiques sans trouble hépatique, sans risque de trouble hépatique, et/ou sans signes ou symptômes d'un trouble hépatique.

12. Procédé selon l'une quelconque des revendications 1-2, dans lequel la quantité d'EZE et/ou d'EZE-Gluc est augmentée d'au moins 50 % par rapport à la valeur témoin ou de référence.
